# EUROPÄISCHE PATENTSCHRIFT

(11) **EP 2 812 650 B1**
(45) Veröffentlichungstag und Bekanntmachung des Hinweises auf die Patenterteilung: **24.07.2019**
(21) Anmeldenummer: 13708647.6
(22) Anmeldetag: 04.02.2013
(51) Int. Cl.: G01B 11/25, A61C 9/00, A61B 5/107

(54) **VERFAHREN ZUM BETREIBEN EINER VORRICHTUNG ZUM ERFASSEN DER DREIDIMENSIONALEN GEOMETRIE VON OBJEKTEN**
METHOD FOR THE OPERATION OF A DEVICE FOR DETECTING THE THREE-DIMENSIONAL GEOMETRY OF OBJECTS
PROCÉDÉ DE FONCTIONNEMENT D'UN DISPOSITIF D'ENREGISTREMENT DE LA GÉOMÉTRIE TRIDIMENSIONNELLE D'OBJETS

(30) Priorität: 06.02.2012 DE 102012100953
(43) Veröffentlichungstag der Anmeldung: 17.12.2014
(73) Patentinhaber: a.tron3d GmbH, 9020 Klagenfurt am Wörthersee (AT)
(72) Erfinder: NOWAK, Christoph, A-1100 Wien (AT); KOINIG, Horst, A-9020 Klagenfurt (AT); JESENKO, Jürgen, A-9584 Finkenstein (AT)
(74) Vertreter: Beer & Partner Patentanwälte KG
(86) Internationale Anmeldenummer: PCT/AT2013/000018
(87) Internationale Veröffentlichungsnummer: WO 2013/116881

(56) Entgegenhaltungen:
- EP-A1- 2 166 303
- EP-A2- 0 250 993
- EP-B1- 0 837 659
- WO-A1-2010/012838
- WO-A2-2009/063088
- DE-A1- 19 636 354
- DE-A1-102007 060 263
- US-A1- 2003 164 952

## Beschreibung

Die Erfindung betrifft ein Verfahren zum Betreiben einer Vorrichtung zum Erfassen der dreidimensionalen Geometrie von Objekten, insbesondere Zähnen, mit einem Handstück, das wenigstens einen Lagesensor zum Erfassen der Änderung der räumlichen Lage des Handstücks und eine optische Einrichtung mit wenigstens einer Kamera zum Aufnehmen von Bildern und mit wenigstens einer Lichtquelle für einen Projektor aufweist.

Eine Vorrichtung zum Erfassen der dreidimensionalen Geometrie von Objekten ist beispielsweise aus der AT 508 563 B bekannt. Der Anwendungsbereich der Erfindung erstreckt sich dabei auf die Aufnahme von digitalen Zahn- und Kieferabdrücken, die Hilfestellung bei der Diagnose, die Überwachung von Zahnbehandlungen sowie die zuverlässige Kontrolle von eingesetzten Implantaten. Neben weiteren Einsatzgebieten im Bereich der Medizin- und Industrietechnik, beispielsweise im Bereich der Endoskopie, können auch Objekte stereometrisch vermessen werden, die schwer zugänglich sind.

Weitere Vorrichtungen und Verfahren zur intraoralen Bildgebung sind beispielsweise aus WO 2010/012838 A1, DE 196 36 354 A1, EP 0 250 993 A2, WO 2009/063088 A2, DE 10 2007 060 263 A1, US 2003/164952 A1, EP 0 837 659 B1 oder EP 2 166 303 A1 bekannt.

Die Verwendung eines Lagesensors ist beispielsweise aus der US 5,661,519 A bekannt.

Aufgabe der Erfindung ist es, solche Vorrichtungen derart zu verbessern, dass sie mit möglichst geringer Stromversorgung zu betreiben sind. Damit soll ein Betrieb mittels eines im Handstück selbst untergebrachten und daher kleinen Energiespeichers ermöglich werden.

Bei einer Vorrichtung zum Ausführen des Verfahrens wird diese Aufgabe dadurch gelöst, dass die optische Einrichtung ausschließlich starr befestigte Teile aufweist und dass ein Mittel zum Erzeugen von Licht der Lichtquelle im Handstück angeordnet ist.

Erfindungsgemäß wird diese Aufgabe bei einem Verfahren der eingangs genannten Art dadurch gelöst, dass vom Lagesensor im Handstück ermittelt wird, wie groß eine Änderung der räumlichen Lage der Vorrichtung ist, und daraus bestimmt wird, wie viele Aufnahmen von der Kamera in einer definierten Zeiteinheit gemacht werden.

Durch die Anordnung des Mittels zum Erzeugen des Lichts direkt im Handstück werden lange optische Wege, beispielweise über Glasfaserkabel oder viele Umlenkspiegel, vermieden. Es wird dabei zwischen der Lichtquelle, also allem was Licht aussenden kann, beispielsweise das Ende eines Glasfaserkabels, und dem Mittel zum Erzeugen des Lichts, beispielsweise einem Laser oder dem Halbleiter einer LED, unterschieden.

Durch den Verzicht auf lange optische Wege kann ein Mittel zum Erzeugen des Lichts mit einer geringeren Leistung verwendet werden um das Objekt ausreichend auszuleuchten, was eine bemerkenswerte Energieersparnis bedeutet.

Die starre Montage aller Elemente der optischen Einrichtung bedeutet, dass es nicht möglich ist, die Optik der Kamera zu fokussieren. Alle Kalibrierungen der optischen Einrichtung erfolgen also im Vorfeld. Dabei ist es insbesondere wichtig eine optimale Einstellung der Blende zu erreichen. Eine kleinere Blende ist dabei gut für eine größere Schärfentiefe, bei größerer Blende wird eine geringere Ausleuchtung für eine ausreichend gute Aufnahme benötigt.

In einer Ausführungsform weist die Vorrichtung eine Einrichtung zum Synchronisieren der Energieversorgung von Lichtquelle und Kamera auf. So werden Kamera und Lichtquelle entsprechend einer bevorzugten Durchführungsform des Verfahrens synchron betrieben. Durch das Pulsen von Licht können punktuell große Leistungen bei verhältnismäßig geringem Energieaufwand erreicht werden. Bei dieser Ausführungsform der Erfindung wird auch auf Seiten der Aufnahme die Energieversorgung unterbrochen. So werden unbeleuchtete Aufnahmen vermieden und weiter Energie eingespart.

In einer weiteren Ausführungsform weist das Handstück wenigstens einen Lagesensor, insbesondere einen Beschleunigungssensor, einen Magnetfeldsensor und/oder einen Neigungssensor auf. Mit diesem wird verfahrensgemäß ermittelt, wie groß die Änderung der räumlichen Lage der Vorrichtung ist, und daraus bestimmt, wie viele Aufnahmen von der Kamera in einer definierten Zeiteinheit gemacht werden sollen. So kann vermieden werden, dass bei geringer Bewegung mehr Bilder von ein und derselben Stelle gemacht werden, als für eine optimale Erfassung der Geometrie notwendig ist.

In diesem Sinne kann in einer bevorzugten Durchführungsform die Bildrate der aufgenommenen Bilder verändert werden, bevorzugt liegt die Bildrate zwischen 1 und 30 Bildern pro Sekunde.

Zusätzlich oder alternativ kann gemäß einer bevorzugten Durchführungsform des Verfahrens die Bildrate auch abhängig davon angepasst werden, ob eine größerer oder geringerer Ladestand eines Energiespeichers bzw. ein größerer oder geringerer möglicher Entladestrom zur Verfügung steht. So können bei größerem Ladestand bzw. Entladestrom mehr Lichtpulse ausgesendet und aufgenommen werden als bei niedrigerem Ladestand bzw. Entladestrom.

In einer möglichen Ausführungsform der Erfindung kann zusätzlich bestimmt werden, wie viele Bilder von einem definierten Bereich erfasst wurden. Aus diesem Wert kann einem aufgenommenen Bereich des Objektes eine Qualität zugeordnet werden, die gegebenenfalls in der 3D-Darstellung der Geometrie des Objektes wiedergegeben werden kann, so dass der Nutzer darauf reagieren kann. Bereiche, von denen nur wenige Daten erfasst wurden, die also eine größere Gefahr von Abweichungen von der Geometrie des Objektes aufweisen, können beispielsweise rot dargestellt werden. Bereiche, in denen die Anzahl der Aufnahmen bereits bei einem für die gewünschte Qualität ausreichenden Wert liegt, können beispielsweise grün dargestellt werden. Weitere Farben für Zwischenstufen sind ebenso denkbar wie für Bereiche, in denen bereits ein optimaler Wert erreicht ist, also weitere Aufnahmen keine wesentliche Verbesserung der erfassten Daten mehr bringen. Man kann natürlich auch nur Bereiche, die eine mindere Qualität haben, einfärben.

Im Sinne einer Energieersparnis kann nach einem zusätzlichen oder alternativen Verfahrensschritt für einen definierten Bereich ermittelt werden, wie viele Aufnahmen von diesem Bereich bereits gemacht wurden und ab Erreichen einer definierten Anzahl von Aufnahmen keine weiteren Aufnahmen dieses Bereiches machen. Diese Maßnahme eignet sich außerdem dazu, die benötigten Verarbeitungsschritte in einer Recheneinheit, welche die aufgenommenen Daten verarbeitet, zu optimieren, bzw. benötigte Rechenleistung einzusparen.

In einer Ausführungsform weist die optische Einrichtung wenigstens einen Projektor zur Projektion von Mustern auf. Die Projektion von Mustern verbessert die Möglichkeiten zur Erfassung der dreidimensionalen Geometrie.

In einer weiteren Ausführungsform überdecken der Feldwinkel der Kamera und der Feldwinkel des Projektors einander zu wenigstens 50%, bevorzugt zu wenigstens 80%, besonders bevorzugt zu wenigstens 90%. Der Feldwinkel ist der kegelförmige Bereich, in dem die Projektion bzw. die Aufnahme erfolgt. Durch eine möglichst große Überschneidung wird ein möglichst großer Anteil der aufgewendeten Energie genutzt.

In einer Ausführungsform weist die Vorrichtung einen gegebenenfalls wieder aufladbaren elektrischen Energiespeicher auf.

Dieser kann als alleinige Energiequelle der Vorrichtung dienen. In diesem Fall ist es sinnvoll, wenn die Vorrichtung weiters einen Datenspeicher oder eine Möglichkeit der kabellosen Datenübertragung aufweist. So kann das Gerät vollkommen frei ohne Kabel bewegt werden. In einer Ausführungsform, bei der die Daten gespeichert werden, ist es zweckmäßig, die spätere Übertragung der Daten, beispielsweise über einen USB-Anschluss, mit einem Aufladen des Energiespeichers zu verbinden.

Bevorzugt ist eine hygienisch bzw. dicht verschließbare Öffnung im Gehäuse zum Austauschen des Energiespeichers vorgesehen. So kann beispielsweise eine Bedienperson nach einer Verwendung des Scanners den zumindest teilweise entleerten Energiespeicher gegen einen vollen tauschen. Alternativ oder zusätzlich kann der Energiespeicher auch nach einer gewissen Anzahl von Ladezyklen und einem tatsächlichen oder zu erwartenden Nachlassen der Leistung des Energiespeichers durch eine Fachkraft ausgetauscht werden.

Alternativ oder zusätzlich kann in einer weiteren bevorzugten Durchführungsform der Erfindung aus dem ermittelten Wert der zur Verfügung stehenden Akkuladung bestimmt werden, ob das Gerät gegebenenfalls mit zwei oder drei oder mehr Kameras betrieben werden soll. So werden für unterschiedliche Leistungen der Stromversorgung bzw. für verschiedene Ladestände unterschiedliche Betriebsmodi geschaffen.

In einer besonders bevorzugten Durchführungsform des Verfahrens werden die von der Kamera erfassten Daten ohne weitere Verarbeitung bzw. Aufbereitung an eine Recheneinheit oder ein Speichermedium weitergeleitet. So wird der Energieaufwand, der sonst für einen Prozessor bzw. Chip, der diese Verarbeitung bzw. Aufbereitung üblicherweise durchführt, vollkommen vermieden. Die weitere Verarbeitung in der Recheneinheit kann wenigstens teilweise in der CPU erfolgen, allerdings hat es sich gezeigt, dass es insbesondere im Hinblick auf die Schnelligkeit der Datenverarbeitung sinnvoll ist, einen Teil der für die Erfassung bzw. Berechnung der dreidimensionalen Geometrie erhobenen Daten in der GPU zu verarbeiten. So ist es möglich die Daten, insbesondere mittels der Kameras aufgenommene zweidimensionale Bilder ohne nennenswerten Zeitverlust direkt in eine dreidimensionale Darstellung auf einem Display bzw. eine auf einem Speichermedium verfügbare Datei (beispielsweise ein 3D-File im STL-Format) umzuwandeln.

Die Vorrichtung kann in einer Ausführungsform ein thermovoltaisches Element aufweisen. Mit diesem kann gemäß einer bevorzugten Durchführungsform des Verfahrens aus der Wärme, welche beim Betrieb entsteht, elektrische Energie gewonnen werden. Diese kann dann zum einen direkt zum Betreiben der Vorrichtung verwendet werden, zum anderen kann aber, insbesondere beim Auskühlen des Gerätes auch ein Energiespeicher mit der gewonnenen Energie gespeist werden.

Weitere bevorzugte Aus- und Durchführungsformen der Erfindung sind Gegenstand der übrigen Unteransprüche.

Die Erfindung wird in der Folge unter Bezugnahme auf die Zeichnungen weiter erläutert.
- Fig. 1: zeigt eine schematisierte Darstellung einer Ausführungsform zum Durchführen der Erfindung und
- Fig. 2: zeigt eine schematische Ansicht der Unterseite einer Ausführungsform zum Durchführen der Erfindung.

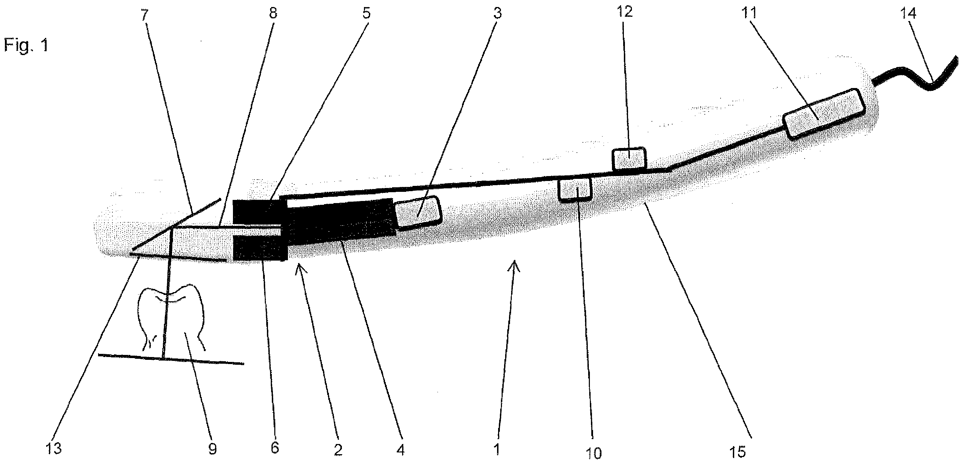

Die Fig. 1 zeigt eine beispielhafte Ausführungsform der Vorrichtung, bestehend aus einem Handstück 1, in welchem sich eine optische Einrichtung 2 befindet, welche eine Lichtquelle 3, einen Projektor 4, eine erste Kamera 5, eine zweite Kamera 6 sowie einen Spiegel 7 enthält. Vor dem Spiegel befindet sich im Gehäuse 15 des Handstücks 1 eine Ausnehmung. Diese ist aus hygienischen Gründen und zum Schutz der im Handstück 1 befindlichen Bauteile mit einer transparenten Abdeckung 13 versehen.

In dieser Ausführungsform ist die Lichtquelle 3 eine LED. Ein Mittel zum erzeugen des Lichts (in der Zeichnung nicht dargestellt) befindet sich in diesem Ausführungsbeispiel also in Form eines Halbleiters direkt in der Lichtquelle 3. Der weitere Verlauf des Lichts innerhalb und außerhalb der Vorrichtung ist durch einen beispielhaften Lichtstrahl 8 dargestellt.

Dieser durchläuft dabei zunächst den Projektor 4. Der Projektor 4 dient dabei der Projektion von Mustern auf das Objekt. Dabei kann es sich, abhängig von der Art der Erfassung der Geometrie, sowohl um regelmäßige Muster, wie beispielsweise Streifen, als auch um unregelmäßige Muster, wie beispielsweise unregelmäßige Punktemuster, handeln.

Nach dem Projektor 4 trifft der Lichtstrahl 8 auf den Spiegel 7 und wird über diesen auf das Objekt 9, dessen Geometrie erfasst werden soll, umgelenkt. Im dargestellten Ausführungsbeispiel handelt es sich bei dem Objekt 9 um einen Zahn. In einer in der Zeichnung nicht dargestellten Ausführungsform, bei der die Lichtquelle 3 und der Projektor 4 bereits in Richtung des Objektes ausgerichtet sind, kann auch auf den Spiegel 7 verzichtet werden.

Die Kameras 5,6 nehmen das auf den Zahn 9 projiziere Muster auf, aus welchem später die Geometrie des Zahns 9 errechnet wird. Gemäß einer bevorzugten Durchführungsform finden alle diesbezüglichen Berechnungen in einer Außerhalb des Handstücks 1 befindlichen Recheneinheit statt, wodurch der Stromverbrauch interner Chipsätze bzw. Prozessoren minimiert wird. Zu dieser Recheneinheit kann die Vorrichtung sowohl physisch mit einem Kabel 14 als auch drahtlos verbunden sein. Im Ausführungsbeispiel ist eine drahtlose Verbindung (beispielsweise Bluetooth oder WLAN) vorgesehen. Zu diesem Zweck befindet sich im Handstück ein Mittel zur drahtlosen Datenübertragung 10, insbesondere ein Sender und gegebenenfalls ein Empfänger. Das dargestellte Kabel 14 ist im eigentlichen Betrieb daher nicht angeschlossen. Kann aber bei niedrigem Ladestand des Energiespeichers beispielsweise als Hilfsstrom angeschlossen werden.

Weiters ist im Handstück 1 ein gegebenenfalls wieder aufladbarer Energiespeicher 11 vorgesehen. Es kann folglich vollständig auf ein Kabel am Handstück 1 verzichtet werden, wodurch optimale Bewegungsfreiheit gegeben ist.

Die Zeichnung zeigt außerdem einen Lagesensor 12. Mit diesem kann ermittelt werden, wie groß die räumliche Bewegung des Handstücks 1 ist. Zu diesem Zweck kann der Lagesensor 12 beispielsweise ein Beschleunigungssensor, ein Erdmagnetfeldsensor oder ein Neigungssensor sein. Kombinationen unterschiedlicher Sensortypen erhöhen dabei die Genauigkeit mit der die Änderung der räumlichen Lage bzw. die Bewegung des Handstücks 1 ermittelt wird.

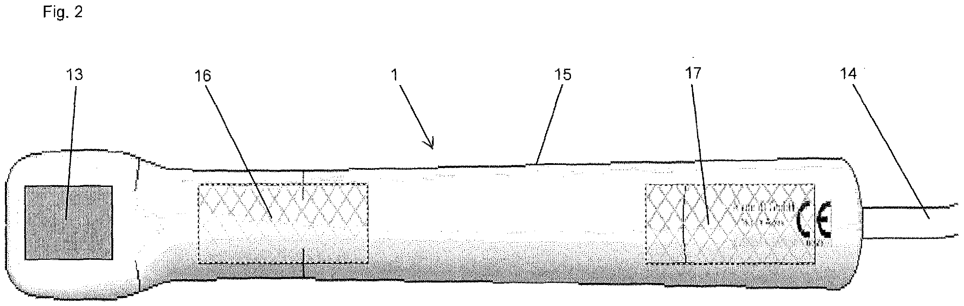

Die Fig. 2 zeigt eine schematische Ansicht der Unterseite auf eine Ausführungsform der Erfindung. Dabei sind zwei Bereiche 16, 17 gezeigt, in denen ein thermovoltaisches Element platziert werden könnte.

Im ersten Bereich 16, wird das thermovoltaische Element direkt an der Unterseite, also der Seite auf welcher sich die Abdeckung 13 befindet, in der Nähe der optischen Einrichtung 2 angeordnet. Dies ist vorteilhaft, da die optische Einrichtung 2, insbesondere der Projektor 4, während des Betriebs am meisten Wärme produziert und diese so mit möglichst geringen Verlusten genutzt werden kann.

Wird das thermovoltaische Element im zweiten Bereich 17 platziert, hat dies den Vorteil, dass es größer dimensioniert werden kann, allerdings wird dann ein Wärmeleiter, der die Wärme von der optischen Einrichtung 2 zum thermovoltaischen Element leitet, notwendig. Auch bei einer Positionierung des thermovoltaischen Elements im zweiten Bereich 17 ist eine Anbringung an der Unterseite des Handstücks 1 sinnvoll, damit eine gemäß einer Ausführungsform nach außen zeigende, Wärme abgebende Seite des thermovoltaischen Elementes nicht von der Hand des Nutzers abgedeckt wird.

## Patentansprüche

1. Verfahren zum Betreiben einer Vorrichtung zum Erfassen der dreidimensionalen Geometrie von Objekten (9), insbesondere Zähnen, mit einem Handstück (1), das wenigstens einen Lagesensor (12) zum Erfassen der Änderung der räumlichen Lage des Handstücks (1) und eine optische Einrichtung (2) mit wenigstens einer Kamera (5, 6) zum Aufnehmen von Bildern und mit wenigstens einer Lichtquelle (3) für wenigstens einen Projektor (4) aufweist, **dadurch gekennzeichnet, dass** vom Lagesensor (12) im Handstück (1) ermittelt wird, wie groß eine Änderung der räumlichen Lage der Vorrichtung ist, und daraus bestimmt wird, wie viele Aufnahmen von der Kamera (5, 6) in einer definierten Zeiteinheit gemacht werden.

2. Verfahren nach Anspruch 1 **dadurch gekennzeichnet, dass** die Kamera (5, 6) synchron zur Lichtquelle (3) des Projektors (4) betrieben wird.

3. Verfahren nach Anspruch 1 oder 2 **dadurch gekennzeichnet, dass** die von der Kamera (5, 6) erfassten Daten ohne weitere Verarbeitung bzw. Aufbereitung an eine Recheneinheit oder ein Speichermedium weitergeleitet werden.

4. Verfahren nach einem der Ansprüche 1 bis 3 **dadurch gekennzeichnet, dass** eine Bildrate der aufgenommenen Bilder zwischen 1 und 30 Bildern pro Sekunde liegt.

5. Verfahren nach einem der Ansprüche 1 bis 4 **dadurch gekennzeichnet, dass** für einen definierten Oberflächenbereich des Objektes (9) ermittelt wird, wie viele Aufnahmen von diesem Bereich bereits gemacht wurden, und dass ab Erreichen einer definierten Anzahl von Aufnahmen keine weiteren Aufnahmen dieses Bereiches mehr gemacht werden.

## Claims

1. A method for operating a device for detecting the three-dimensional geometry of objects (9), in particular teeth, with a handpiece (1), which comprises at least one position sensor (12) for detecting the change in the spatial position of the handpiece (1) and an optical means (2) with at least one camera (5, 6) for capturing images and with at least one light source (3) for at least one projector (4), **characterised in that** it is ascertained by the position sensor (12) in the handpiece (1), how big a change is in the spatial position of the device, and on this basis a determination is made, how many shots the camera (5, 6) takes within a defined unit of time.

2. The method according to claim 1, **characterised in that** the camera (5, 6) is operated synchronously with the light source (3) of the projector (4).

3. The method according to claim 1 or 2, **characterised in that** the data recorded by the camera (5, 6) is forwarded to a computing unit or a storage medium without any further processing or preparation.

4. The method according to one of claims 1 to 3, **characterised in that** a frame rate of the images captured lies between 1 and 30 frames per second.

5. The method according to one of claims 1 to 4, **characterised in that** it is ascertained for a defined surface region of the object (9), how many shots have already been taken of this region and that once a defined number of shots has been reached, no further shots of this region are taken.

## Revendications

1. Procédé pour exploiter un dispositif pour détecter la géométrie tridimensionnelle d'objets (9), en particulier de dents, avec une pièce à main (1) qui présente au moins un capteur de position (12) pour détecter la variation de la position dans l'espace de la pièce à main (1) et une installation optique (2) avec au moins une caméra (5, 6) pour enregistrer des images et avec au moins une source lumineuse (3) pour au moins un projecteur (4), **caractérisé en ce que** le capteur de position (12) dans la pièce à main (1) détermine l'ampleur d'une variation de la position du dispositif dans l'espace et qu'à partir de cela, on détermine le nombre de prises de vue qu'on a pris par la caméra (5, 6) dans une unité de temps définie.

2. Procédé selon la revendication 1, **caractérisé en ce que** la caméra (5, 6) est exploitée de manière synchrone avec la source lumineuse (3) du projecteur (4).

3. Procédé selon la revendication 1 ou 2, **caractérisé en ce que** des données collectées par la caméra (5, 6) sont transmises sans autre traitement ou préparation à une unité de calcul ou un support de stockage.

4. Procédé selon l'une des revendications 1 à 3, **caractérisé en ce qu'**un débit d'image des images enregistrées est compris entre 1 et 30 images par seconde.

5. Procédé selon l'une des revendications 1 à 4, **caractérisé en ce que**, pour une zone de surface définie de l'objet (9), on détermine le nombre de prises de vue qu'on a déjà pris de cette zone et qu'à l'atteinte d'un nombre de prises de vue défini, on n'effectue plus d'autres prises de vue de cette zone.
